# EUROPEAN PATENT SPECIFICATION

(11) **EP 3 127 437 B1**
(45) Date of publication and mention of the grant of the patent: **05.12.2018**
(21) Application number: 14731531.1
(22) Date of filing: 27.03.2014
(51) Int. Cl.: A24F 47/00, A61M 11/04, A61M 15/06, H05B 3/44

(54) **ELECTRONIC CIGARETTE**
ELEKTRONISCHE ZIGARETTE
CIGARETTE ÉLECTRONIQUE

(43) Date of publication of application: 08.02.2017
(73) Proprietor: Shenzhen Smoore Technology Limited, Shenzhen, Guangdong 518102 (CN)
(72) Inventor: CHEN, Zhiping, Shenzhen Guangdong 518102 (CN)
(74) Representative: Cabinet Laurent & Charras
(86) International application number: PCT/CN2014/074186
(87) International publication number: WO 2015/143666

(56) References cited:
- WO-A1-2014/012906
- CA-A1- 2 878 951
- CA-A1- 2 878 973
- CN-A- 101 843 368
- CN-A- 101 843 368
- CN-A- 103 960 782
- CN-C- 100 593 982
- CN-U- 203 087 527
- CN-U- 203 523 811
- CN-Y- 201 430 916
- JP-A- 2012 029 633
- US-A1- 2012 279 512
- US-A1- 2013 276 804
- US-A1- 2013 306 065
- US-A1- 2013 312 776

## Description

### FIELD OF THE INVENTION

The present invention relates to an electronic cigarette.

### BACKGROUND OF THE INVENTION

Electronic cigarette, also known as virtual cigarette, electronic atomizers, is a substitute of the cigarette for smoking cessation. The electronic cigarette has a similar appearance and taste as the cigarette, but it generally does not contain harmful ingredients of the cigarettes, such as tar, suspended particles, and so on.

The electronic cigarette is usually composed of an atomizer and a power source. The atomizer plays important roles in the perceived quality of the vapor. A conventional heating element of the atomizer is a spring-like resistance wire wrapped around a wicking material. When activated, the resistance wire quickly heats up thus turning the liquid absorbed by the wicking material into a vapor, which is then inhaled by the user.

However, the wicking material of the conventional electronic cigarette has a relatively small volume and surface area, which results in a less absorption of liquid and a poor atomization effect. Other configurations are disclosed for example in documents CA2878973A1, US2013/306065A1, CN101843368, US2012/279512, CA2878951A1, US2013/312776A1 and US2013/276804, which present drawbacks regarding the liquid flow, and thus the atomization effect.

### SUMMARY OF THE INVENTION

In view of this, it is necessary to provide an electronic cigarette with better atomization effect.

An electronic cigarette according to the invention includes: a housing; a mouthpiece located at an end of the housing, the mouthpiece defining an air outlet thereof; an atomizing assembly received in the housing, the atomizing assembly includes: a liquid reservoir for storing liquid, the liquid reservoir defining a channel therethrough communicated with the air outlet; a liquid absorption element connected to the liquid reservoir, the liquid absorption element being configured to absorb the liquid; and a heating element connected to the liquid absorption element, the heating element being configured to atomize the liquid absorbed by the liquid absorption element into atomized gas, the atomized gas entering the channel; and a power source received in the housing and connected to the atomizing assembly, the power source being configured to provide power for the heating element. The invention is defined by the appended claim 1.

In one embodiment, the liquid absorption element is made of porous ceramic.

In one example, the liquid absorption element is received in the channel, the liquid absorption element defines an atomizing passage therethrough communicated with the channel, the heating element is received in the atomizing passage.

In one embodiment, heating element is a spiral tubular heating wire, both ends of the heating wire are connected to the power source via wires.

In one embodiment, the liquid reservoir is filled with a storage medium.

According to the invention, the liquid absorption element is located at an end of the liquid reservoir, the liquid absorption element defines an atomizing passage therethrough communicated with the channel, the heating element is received in the atomizing passage.

According to the invention, the electronic cigarette further includes a reservoir cover positioned between the liquid reservoir and the liquid absorption element, wherein the reservoir cover defines a liquid conduction channel and an airflow channel, the liquid enters the liquid absorption element via the liquid conduction channel, the airflow channel is communicated with the channel, the liquid absorption element defines an atomizing passage therethrough communicated with the channel, the heating element is received in the atomizing passage.

In one example, the liquid absorption element is located at an end of the liquid reservoir, the liquid absorption element defines an atomizing passage therethrough communicated with the channel, the heating element is located at an end of the liquid absorption element away from the liquid reservoir.

In one example, the heating element is a planar spiral heating wire, both ends of the heating wire are connected to the power source via wires.

According to the invention, the liquid absorption element is located at an end of the liquid reservoir, the liquid absorption element defines an atomizing passage therethrough communicated with the channel, the heating element is a heating coating coated on an inner sidewall of the atomizing passage.

Another electronic cigarette includes: a housing; a mouthpiece located at an end of the housing, the mouthpiece defining an air outlet thereof; an atomizing assembly received in the housing, the atomizing assembly includes: a liquid absorption element configured to absorb liquid, the liquid absorption element defining an atomizing passage therethrough communicated with the air outlet; and a heating element connected to the liquid absorption element, the heating element being configured to atomize the liquid absorbed by the liquid absorption element into atomized gas, the atomized gas entering the channel; and a power source received in the housing and connected to the atomizing assembly, the power source being configured to provide power for the heating element.

In one embodiment, the liquid absorption element is made of porous ceramic.

In one embodiment, the heating element is spiral tubular heating wire, both ends of the heating wire are connected to the power source via wires.

According to the invention, the heating element is a heating coating coated on an inner sidewall of the atomizing passage.

In one example, the heating element is a planar spiral heating wire located at an end of the liquid absorption element away from the mouthpiece.

Compared with the prior art, the liquid absorption element of the electronic cigarette has a greater area, it has a greater contact area with the heating element, which results in a better atomizing effect.

### BRIEF DESCRIPTION OF THE DRAWINGS

The components in the drawings are not necessarily drawn to scale, the emphasis instead being placed upon clearly illustrating the principles of the present disclosure. Moreover, in the drawings, like reference numerals designate corresponding parts throughout the views.
FIG. 1 is a exploded perspective view of an example of an electronic cigarette;
FIG. 2 is a cross-sectional view of the electronic cigarette of FIG. 1;
FIG. 3 is a exploded perspective view of an example of an electronic cigarette;
FIG. 4 is a cross-sectional view of the electronic cigarette of FIG. 3;
FIG. 5 is a exploded perspective view of an electronic cigarette according to the invention;
FIG. 6 is a cross-sectional view of the electronic cigarette of FIG. 5;
FIG. 7 is a exploded perspective view of a fourth embodiment of an electronic cigarette;
FIG. 8 is a cross-sectional view of the electronic cigarette of FIG. 7;
FIG. 9 is a exploded perspective view of an example of an electronic cigarette;
FIG. 10 is a cross-sectional view of the electronic cigarette of FIG. 9;
FIG. 11 is a exploded perspective view of an example of an electronic cigarette;
FIG. 12 is a cross-sectional view of the electronic cigarette of FIG. 11;
FIG. 13 is a exploded perspective view of an example of an electronic cigarette; and
FIG. 14 is a cross-sectional view of the electronic cigarette of FIG. 13.

### DETAILED DESCRIPTION OF THE EMBODIMENTS

Reference will now be made to the drawings to describe, in detail, embodiments of the present electronic cigarette. It should be noted that references to "an" or "one" embodiment in this disclosure are not necessarily to the same embodiment, and such references mean at least one.

Unless the context clearly requires otherwise, throughout the description and the claims, the words "comprise," "comprising," and the like are to be construed in an inclusive sense as opposed to an exclusive or exhaustive sense; that is to say, in the sense of "including, but not limited to." Words using the singular or plural number also include the plural or singular number respectively. Additionally, the words "herein,""above," "below" and words of similar import, when used in this application, shall refer to this application as a whole and not to any particular portions of this application. When the claims use the word "or" in reference to a list of two or more items, that word covers all of the following interpretations of the word: any of the items in the list, all of the items in the list and any combination of the items in the list.

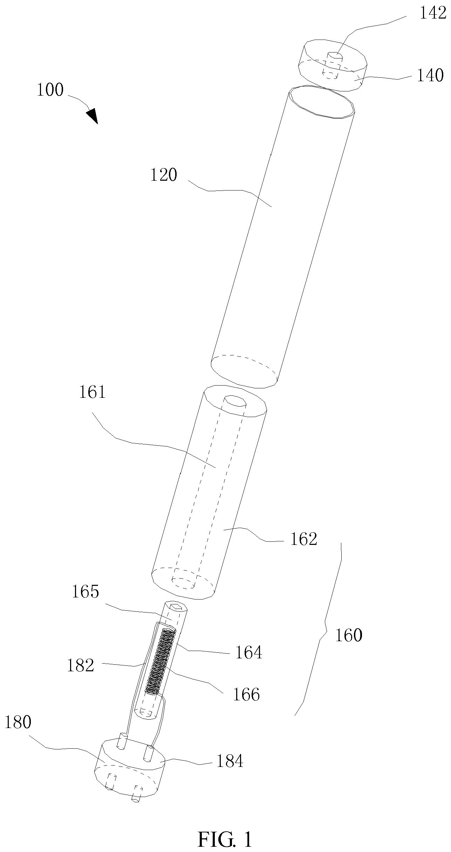

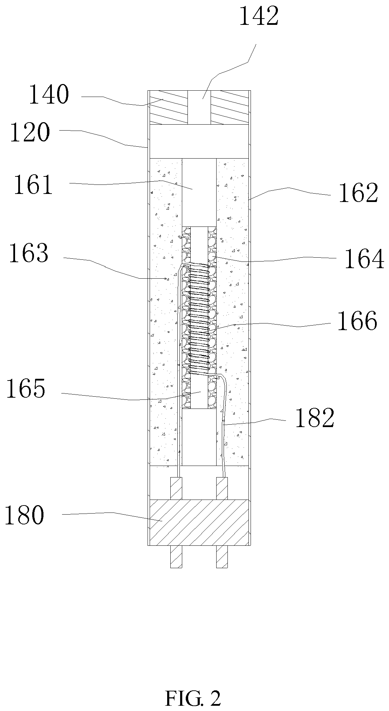

Referring to FIG. 1 and FIG. 2, a first example of an electronic cigarette 100 includes a housing 120, a mouthpiece 140, an atomizing assembly 160, and a power source 180.

The housing 120 is substantially a hollow elongated cylinder. The mouthpiece 140 is located at an end of the housing 120, and the atomizing assembly 160 and the power source 180 are received inside the housing 120. It is to be understood that, the housing 120 may have other shapes, such as rectangular or the like.

The mouthpiece 140 has thread on its outer periphery, and the mouthpiece 140 is threadedly fixed at the top of the housing 120. The mouthpiece 140 defines an air outlet 142 at a center thereof.

The atomizing assembly 160 includes a liquid reservoir 162, a liquid absorption element 164, and a heating element 166.

The liquid reservoir 162 is substantially a hollow tube and is used for storing liquid. The liquid reservoir 162 internally defines a substantially cylindrical channel 161 along an axis thereof. The channel 161 is aligned with the air outlet 142. The liquid reservoir 162 is filled with a storage medium 163 for storing liquid. The storage medium 163 can be made of fibers, preferably modified fibers, which can remove the odor of the liquid, so as not to affect the taste of the smoke.

The liquid absorption element 164 is connected to the liquid reservoir 162. In the illustrated embodiment, the liquid absorption element 164 is shaped as a tube mating with the channel 161. Accordingly, the liquid absorption element 164 can be inserted into the channel 161 of the liquid reservoir 162 and be in contact with the storage medium 163. The liquid absorption element 164 is made of rigid porous material materials with liquid storage and heat-resisting features, such as silicone, porous ceramics, and the like. Accordingly, the liquid from the storage medium 163 can be uniformly dispersed in the interior and surface of the liquid absorption element 164 by capillary action. The liquid absorption element 164 defines an internal atomizing passage 165 in an axial direction communicated with the channel 161.

The heating element 166 is connected to the liquid absorption element 164. In the illustrated example, the heating element 166 is a substantially spiral tubular heating wire received in the atomizing passage 165. The heating element 166 extends in a same direction as that of the atomizing passage 165. The conventional heating wire is laterally positioned in an air flow passage of the conventional electronic cigarette. Due to the small diameter of the air flow passage, only a small winding number of the heating wire can be arranged. However, in the illustrated example, the heating element 166 extends in a same direction as that of the atomizing passage 165, such that the winding number of the heating wire can be greatly increased, thereby improving the heating area and the atomizing effect. The heating element 166 is made of flexible metals or alloys, preferably nichrome wire. Since the heating element 166 has certain flexibility, and the liquid absorption element 164 is made of rigid material, the heating element 166 can therefore be fixed directly in the atomizing passage 165 without the aim of a support element, thus simplifying the structure. Both ends of the heating element 166 are coupled to the power source 180 via two wires 182. When the heating element 166 is powered, the liquid absorption element 164 can be heated by the heating element 166, such that the liquid stored inside the liquid absorption element 164 will be uniformly heated and atomized into uniform vapor particles (i.e. smoke). The smoke enters the channel 161 through the atomizing passage 165, and finally inhaled by the user via the air outlet 142.

The power source 180 includes an electrode holder 184 and a battery (not shown). Both ends of the heating element 166 are coupled to the electrode holder 184 of the power source 180 via two wires 182. The battery is used for providing power for the heating element 166. It is to be understood that, the power source 180 may also include a sensor, an indicator, etc.

Compared with the prior art, the liquid absorption element 164 of the electronic cigarette has a greater area, it has a greater contact area with the heating element 166, which results in a better atomizing effect.

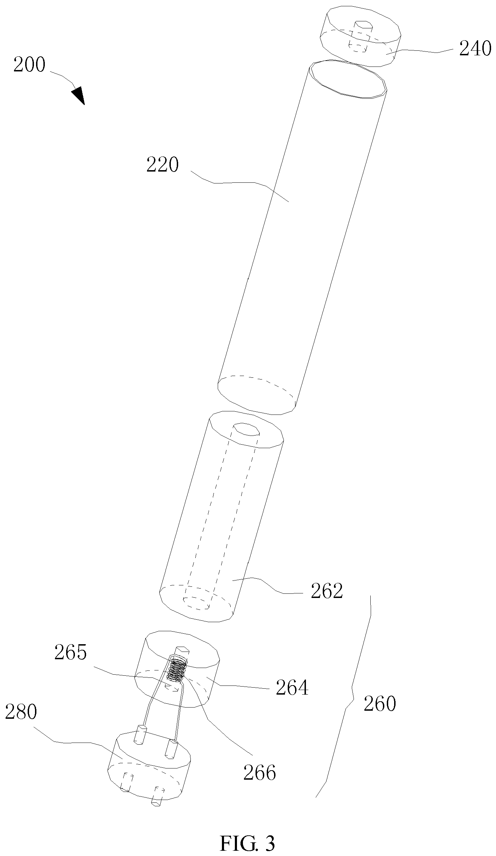

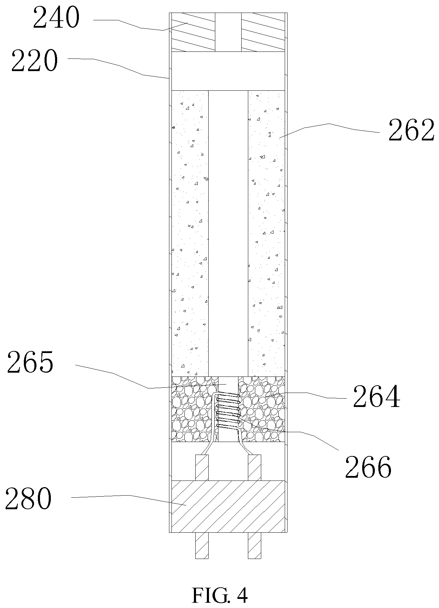

Referring to FIG. 3 and FIG. 4, a second example of an electronic cigarette 200 is similar to the electronic cigarette 100 of the first embodiment. The electronic cigarette 200 includes a housing 220, a mouthpiece 240, an atomizing assembly 260, and a power source 280. The difference lies in that: the liquid absorption element 264 is shaped as tube matching with the housing 220. The liquid absorption element 264 is received in the housing 220 and located at an end of the liquid reservoir 262. The liquid from the liquid reservoir 262 can be uniformly dispersed in the interior and surface of the liquid absorption element 264 by capillary action. The liquid absorption element 264 defines an internal atomizing passage 265 in an axial direction communicated with the channel 261. The heating element 266 is a substantially spiral tubular heating wire received in the atomizing passage 265. Compared with the first embodiment, the liquid absorption element 264 has a larger diameter, thus it can absorb or restore more liquid.

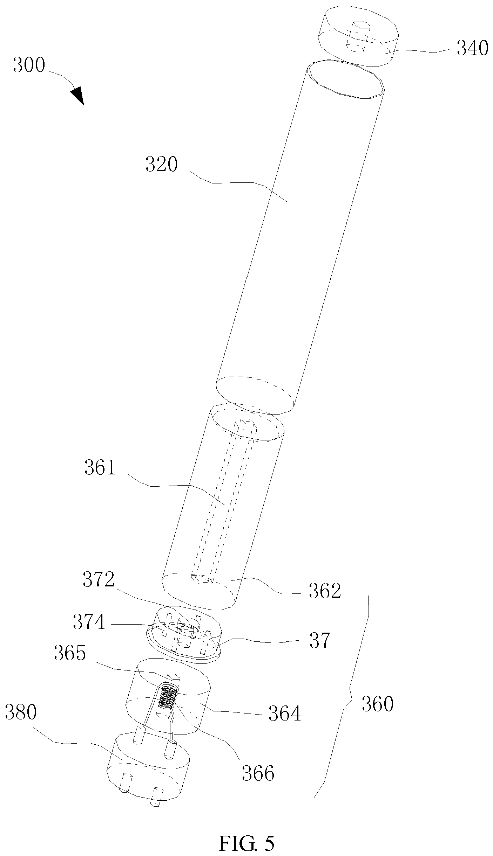

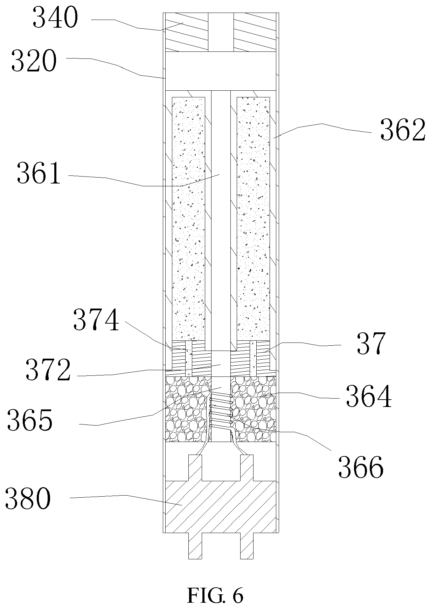

Referring to FIG. 5, and FIG. 6, an electronic cigarette 300 according to the invention, is similar to the electronic cigarette 200 of the second example. The electronic cigarette 300 includes a housing 320, a mouthpiece 340, an atomizing assembly 360, and a power source 380. The difference lies in that: the electronic cigarette 300 further includes a reservoir cover 37 positioned between the liquid reservoir 362 and the liquid absorption element 364. The reservoir cover 37 is shaped as a round cover and is located at an end of the liquid reservoir 362 to seal the liquid reservoir 362. The reservoir cover 37 defines an airflow channel 372 in a middle portion thereof communicated with the channel 361. The reservoir cover 37 further defines four liquid conduction channels 374 evenly distributed around the airflow channel 372. The liquid in the liquid reservoir 362 can flow into the liquid absorption element 364 via the four liquid conduction channels 374. The liquid absorption element 364 defines an internal atomizing passage 365 in an axial direction communicated with the airflow channel 372. The heating element 366 is received in the atomizing passage 365. Compared with the second embodiment, the liquid of the third embodiment can flow into the liquid absorption element 364 via the liquid conduction channels 374, such that the flow of liquid can be more accurately controlled. It should be noted that, the number of the liquid conduction channels 374 can be three, five or more.

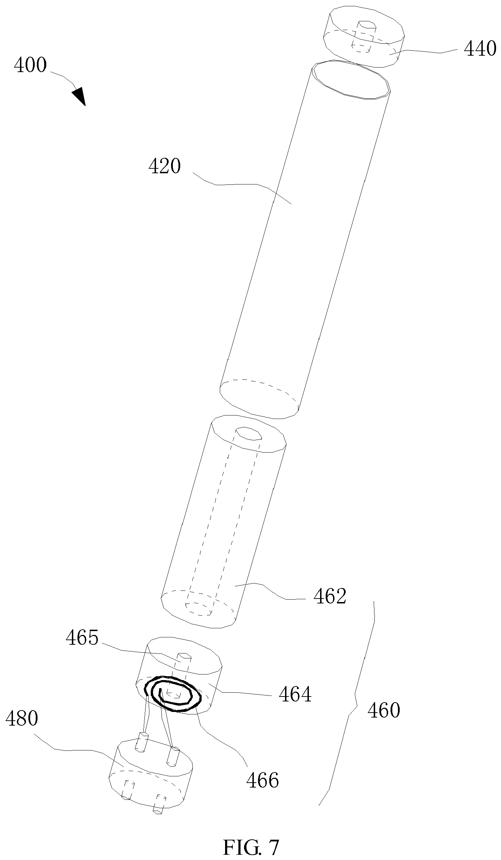

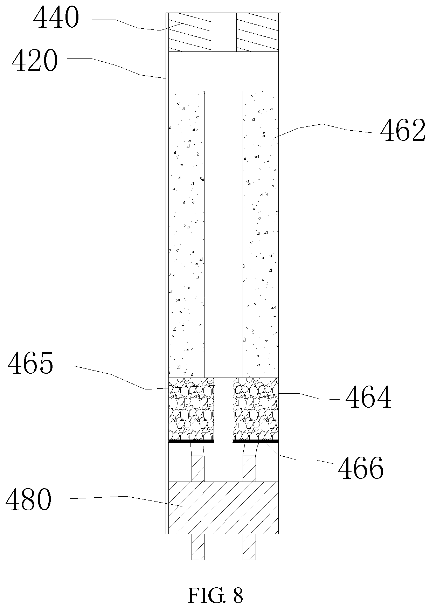

Referring to FIG. 7 and FIG. 8, another example of an electronic cigarette 400 is similar to the electronic cigarette 200 of the second example. The electronic cigarette 400 includes a housing 420, a mouthpiece 440, an atomizing assembly 460, and a power source 480. The difference lies in that: the heating element 466 is located at an end of the liquid absorption element 464 away from the liquid reservoir 462. The heating element 466 is a planar spiral heating wire, and both ends of the heating wire are connected to the power source 480 via wires. Compared with the second example, the heating element 466 of the fourth embodiment has a greater area and a better heating effect.

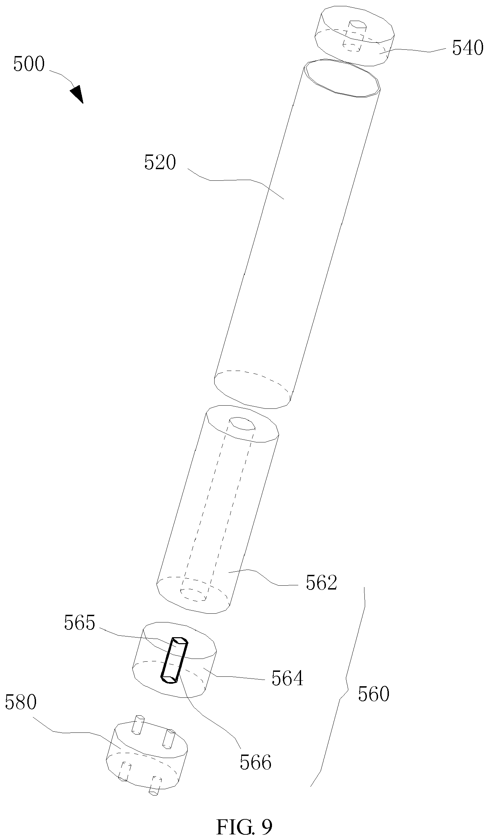

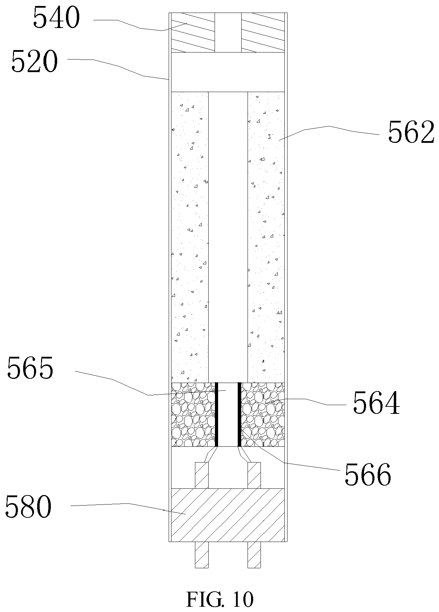

Referring to FIG. 9 and FIG. 10, another example of an electronic cigarette 500 is similar to the electronic cigarette 200 of the second example. The electronic cigarette 500 includes a housing 520, a mouthpiece 540, an atomizing assembly 560, and a power source 580. The difference lies in that: the heating element 566 is a heating coating coated on an inner sidewall of the atomizing passage 565. The heating coating can be a metal heating film or non-metallic heating film. The heating coating can be coated on the inner sidewall of the atomizing passage 565 by printing and is coupled to the power source 380 via wires. Compared with the second example, besides heat conduction through the air convection, the heating coating can conduct heat using far infrared radiation due to the planar heating, thus it can save power compared to other heating methods.

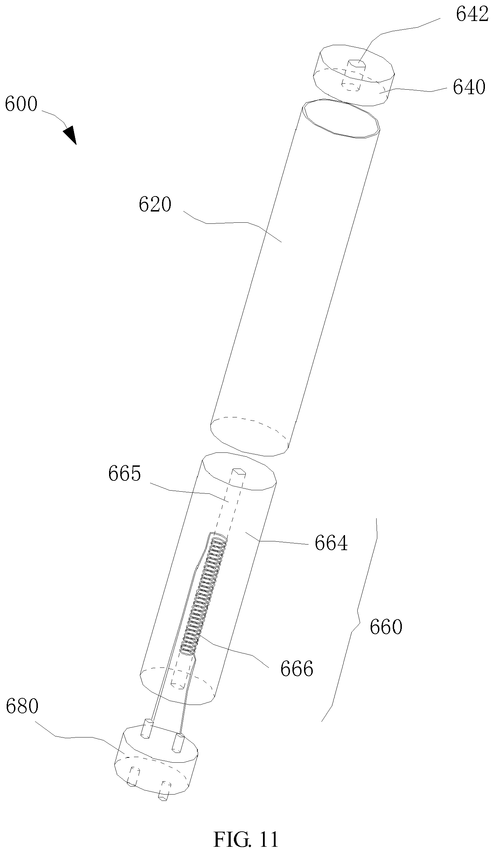

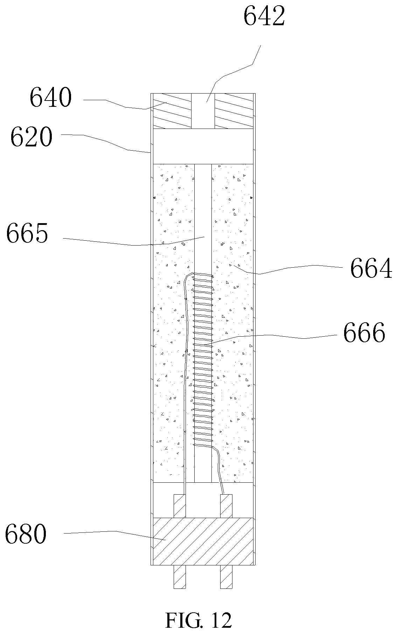

Referring to FIG. 11 and FIG. 12, another example of an electronic cigarette 600 is similar to the electronic cigarette 100 of the first example. The electronic cigarette 600 includes a housing 620, a mouthpiece 640, an atomizing assembly 660, and a power source 680. The difference lies in that: the atomizing assembly 660 includes a liquid absorption element 664 and a heating element 666 but without the liquid reservoir. Since the liquid absorption element 664 itself has a feature of absorption, it can be used to restore liquid directly. Since the liquid reservoir is omitted, the atomizing passage 665 of the liquid absorption element 664 is communicated with the air outlet 642 directly. Compared with the first embodiment, the electronic cigarette 600 of the sixth embodiment has a simple structure.

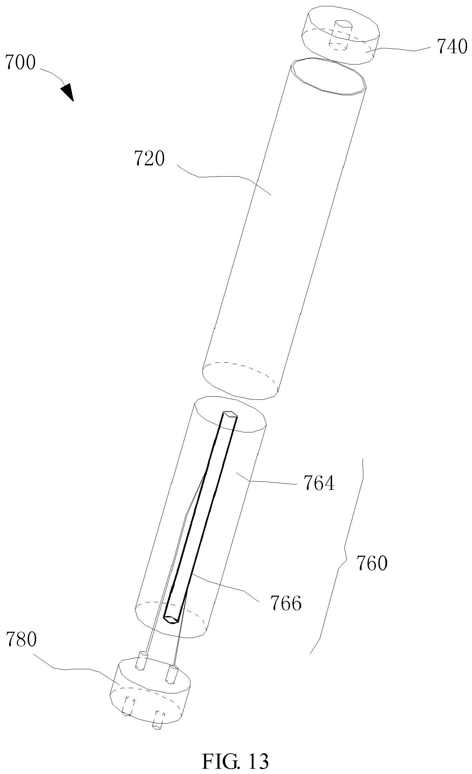

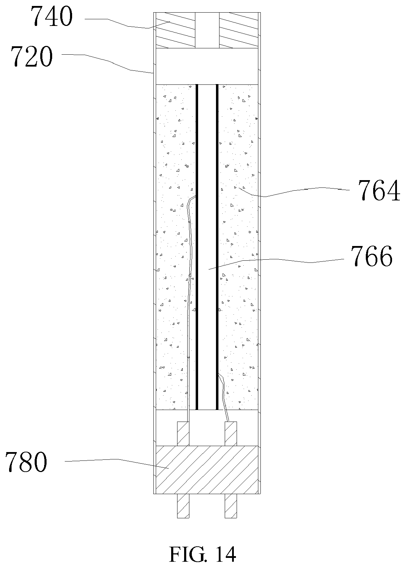

Referring to FIG. 13 and FIG. 14, another example of an electronic cigarette 700 is similar to the electronic cigarette 600 of the previous example. The electronic cigarette 700 includes a housing 720, a mouthpiece 740, an atomizing assembly 760, and a power source 780. The difference lies in that: the heating element 766 is a heating coating coated on an inner sidewall of the atomizing passage 765. The heating coating has the same structure and feature as that of the electronic cigarette 500, which has a better heating effect and can save power. It should be understood that, in alternative embodiment, the heating element 766 can be planar spiral heating wire located at an end of the liquid absorption element 764 away from the mouthpiece 740.

Although the present invention has been described with reference to the examples thereof and the best modes for carrying out the present invention, it is apparent to those skilled in the art that a variety of modifications and changes may be made without departing from the scope of the present invention, which is intended to be defined by the appended claims.

## Claims

1. An electronic cigarette, comprising:
- a housing (320);
- a mouthpiece (340) located at an end of the housing, the mouthpiece (340) defining an air outlet thereof;
- an atomizing assembly (360) received in the housing (320), the atomizing assembly (360) comprising:
. a liquid reservoir (362) for storing liquid, the liquid reservoir (362) defining a channel (361) therethrough communicated with the air outlet;
. a liquid absorption element (364) connected to the liquid reservoir (362), the liquid absorption element (364) being configured to absorb the liquid; and
. a heating element (366) connected to the liquid absorption element (364), the heating element (366) being configured to atomize the liquid absorbed by the liquid absorption element (364) into atomized gas, the atomized gas entering the channel (361); and
- a power source (380) received in the housing (320) and connected to the atomizing assembly (360), the power source (380) being configured to provide power for the heating element (366);
**characterized in that** the electronic cigarette further comprises:
- a reservoir cover (37) located at an end of the liquid reservoir (362) and positioned between the liquid reservoir (362) and the liquid absorption element (364), wherein the reservoir cover (37) defines a liquid conduction channel (374) and an airflow channel (372), the liquid entering the liquid absorption element (364) via the liquid conduction channel (374), the airflow channel (372) communicating with the channel (361), the liquid absorption element (364) defining an atomizing passage (365) therethrough communicating with the airflow channel (372), the heating element (366) being received in the atomizing passage (365).

2. The electronic cigarette according to claim 1, wherein the liquid absorption element (364) is made of porous ceramic.

3. The electronic cigarette according to claim 1, wherein the heating element (366) is a spiral tubular heating wire, both ends of the heating wire are connected to the power source (380) via wires.

4. The electronic cigarette according to claim 1, wherein the liquid reservoir is filled with a storage medium.

5. The electronic cigarette according to claim 1, wherein the heating element (366) is a heating coating coated on an inner sidewall of the atomizing passage (365).

6. The electronic cigarette according to claim 1, wherein the reservoir cover (37) is shaped as a round cover and is located at an end of the liquid reservoir (362) to seal the liquid reservoir (362), the reservoir defining the airflow channel (372) in a middle portion thereof, and defining four liquid conduction channels (374) evenly distributed around the airflow channel (372).

## Patentansprüche

1. Eine elektronische Zigarette, umfassend:
- ein Gehäuse (320);
- ein Mundstück (340), das an einem Ende des Gehäuses angeordnet ist, wobei das Mundstück (340) einen Luftauslass davon definiert;
- eine Zerstäubungseinheit (360), die in dem Gehäuse (320) aufgenommen ist, wobei die Zerstäubungseinheit (360) folgendes umfasst:
• einen Flüssigkeitsbehälter (362) zum Speichern von Flüssigkeit, wobei der Flüssigkeitsbehälter (362) einen ihn durchlaufenden Kanal (361) definiert, der mit dem Luftauslass verbunden ist;
• ein Flüssigkeitsaufnahmeelement (364), das mit dem Flüssigkeitsspeicher (362) verbunden ist, wobei das Flüssigkeitsaufnahmeelement (364) konfiguriert ist, um die Flüssigkeit aufzunehmen; und
• ein Heizelement (366), das mit dem Flüssigkeitsaufnahmeelement (364) verbunden ist, wobei das Heizelement (366) konfiguriert ist, um die von dem Flüssigkeitsaufnahmeelement (364) absorbierte Flüssigkeit in atomisiertes Gas zu zerstäuben, wobei das zerstäubte Gas in den Kanal (361) eintritt; und
- eine Stromquelle (380), die in dem Gehäuse (320) aufgenommen und mit der Zerstäubungseinheit (360) verbunden ist, wobei die Stromquelle (380) konfiguriert ist, um Strom für das Heizelement (366) bereitzustellen;
**dadurch gekennzeichnet, dass** die elektronische Zigarette ferner umfasst:
- einen Speicherhohlraum (37), der an einem Ende des Flüssigkeitsbehälters (362), zwischen dem Flüssigkeitsbehälter (362) und dem Flüssigkeitsaufnahmeelement (364) angeordnet ist, wobei die Speicherabdeckung (37) einen Flüssigkeitskanal (374) und einen Luftströmungskanal (372) definiert, wobei die Flüssigkeit über den Flüssigkeitsleitkanal (374) in das Flüssigkeitsaufnahmeelement (364) eintritt, der Luftströmungskanal (372) mit dem Kanal (361) in Verbindung steht, wobei das Flüssigkeitsaufnahmeelement (364) eine Zerstäubungspassage (365) definiert, der mit dem Luftströmungskanal (372) in Verbindung steht, und wobei das Heizelement (366) in der Zerstäubungspassage (365) aufgenommen ist.

2. Elektronische Zigarette nach Anspruch 1, wobei das Flüssigkeitsaufnahmeelement (364) aus poröser Keramik gefertigt ist.

3. Elektronische Zigarette nach Anspruch 1, wobei das Heizelement (366) ein spiralförmiger rohrförmiger Heizdraht ist, wobei beide Enden des Heizdrahtes über Drähte mit der Stromquelle (380) verbunden sind.

4. Elektronische Zigarette nach Anspruch 1, wobei der Flüssigkeitsbehälter mit ein Speichermedium gefüllt ist.

5. Elektronische Zigarette nach Anspruch 1, wobei das Heizelement (366) eine Heizschicht ist, mit der die innere Seitenwand des Zerstäubungskanals (365) beschichtet ist.

6. Elektronische Zigarette nach Anspruch 1, wobei die Behälterabdeckung (37) als runde Abdeckung ausgebildet ist und an einem Ende des Flüssigkeitsbehälters (362) angeordnet ist, um den Flüssigkeitsbehälter (362) abzudichten, wobei der Behälter den Luftkanal (372) in einem Mittelabschnitt davon definiert und vier Flüssigkeitsleitkanäle (374) definiert, die gleichmäßig um den Luftströmungskanal (372) verteilt sind.

## Revendications

1. Cigarette électronique comprenant :
- un boîtier (320) ;
- un embout buccal (340) situé à une extrémité du boîtier, l'embout buccal (340) définissant une sortie d'air de celui-ci ;
- un ensemble d'atomisation (360) reçu dans le boîtier (320), l'ensemble d'atomisation (360) comprenant :
- un réservoir de liquide (362) pour stocker du liquide, le réservoir de liquide (362) définissant un canal (361) le traversant en communication avec la sortie d'air ;
- un élément d'absorption de liquide (364) connecté au réservoir de liquide (362), l'élément d'absorption de liquide (364) étant configuré pour absorber le liquide ; et
- un élément chauffant (366) relié à l'élément d'absorption de liquide (364), l'élément chauffant (366) étant configuré pour atomiser le liquide absorbé par l'élément d'absorption de liquide (364) en un gaz atomisé, le gaz atomisé pénétrant dans le canal (361) ; et
- une source d'alimentation en énergie (380) reçue dans le boîtier (320) et connectée à l'ensemble d'atomisation (360), la source d'alimentation en énergie (380) étant configurée pour fournir de l'énergie à l'élément chauffant (366) ;
**caractérisée en ce que** la cigarette électronique comprend en outre :
- un couvercle de réservoir (37) situé à une extrémité du réservoir de liquide (362) et positionné entre le réservoir de liquide (362) et l'élément d'absorption de liquide (364), dans laquelle le couvercle de réservoir (37) définit un canal de conduction de liquide (374) et un canal de circulation d'air (372), le liquide entrant dans l'élément d'absorption de liquide (364) par le canal de conduction de liquide (374), le canal de circulation d'air (372) communiquant avec le canal (361), l'élément d'absorption de liquide (364) définissant un passage d'atomisation (365) le traversant en communication avec le canal d'écoulement d'air (372), l'élément chauffant (366) étant reçu dans le passage d'atomisation (365).

2. Cigarette électronique selon la revendication 1, dans laquelle l'élément d'absorption de liquide (364) est constitué de céramique poreuse.

3. Cigarette électronique selon la revendication 1, dans laquelle l'élément chauffant (366) est un fil chauffant tubulaire en spirale, les deux extrémités du fil chauffant étant connectées à la source d'alimentation en énergie (380) par l'intermédiaire de fils.

4. Cigarette électronique selon la revendication 1, dans laquelle le réservoir de liquide est rempli d'un support de stockage.

5. Cigarette électronique selon la revendication 1, dans laquelle l'élément chauffant (366) est un revêtement chauffant appliqué en revêtement sur une paroi latérale interne du passage d'atomisation (365).

6. Cigarette électronique selon la revendication 1, dans laquelle le couvercle de réservoir (37) a la forme d'un couvercle rond et est situé à une extrémité du réservoir de liquide (362) pour sceller le réservoir de liquide (362), le réservoir définissant le canal de circulation d'air (372) dans une partie médiane de celui-ci, et définissant quatre canaux de conduction de liquide (374) répartis uniformément autour du canal de circulation d'air (372).
